# EUROPÄISCHE PATENTSCHRIFT

(11) **EP 0 532 937 B1**
(45) Veröffentlichungstag und Bekanntmachung des Hinweises auf die Patenterteilung: **30.10.1996**
(21) Anmeldenummer: 92114335.0
(22) Anmeldetag: 21.08.1992
(51) Int. Cl.: A61F 13/15

(54) **Decklage zur Verwendung in einem absorbierenden Hygieneartikel und Verfahren zur Herstellung dieser Decklage**
Topsheet for an absorbant sanitary article and method of producing this topsheet
Feuille supérieure pour un article d'hygiène absorbant et procédé de fabrication de cette feuille supérieure

(30) Priorität: 21.08.1991 JP 234124/91
(43) Veröffentlichungstag der Anmeldung: 24.03.1993
(73) Patentinhaber: UNI-CHARM CORPORATION, Kawanoe-shi Ehime-ken (JP)
(72) Erfinder: Yamamoto, Masamitsu, Kawanoe-shi, Ehime-ken (JP); Murakami, Masaki, Kawanoe-shi, Ehime-ken (JP)
(74) Vertreter: Sperling, Rüdiger, Dipl.-Ing.

(56) Entgegenhaltungen:
- EP-A- 0 018 020
- EP-A- 0 156 471
- EP-A- 0 205 286
- DE-A- 3 723 404

## Beschreibung

### DER ERFINDUNG ZUGRUNDELIEGENDER STAND DER TECHNIK

Die vorliegende Erfindung betrifft eine Decklage zur Verwendung in einem Hygieneartikel zur absorbierenden Abführung von Körperflüssigkeiten und ein Herstellungsverfahren der Decklage.

Ein Hygieneartikel zur absorbierenden Abführung von Körperflüssigkeiten, wie etwa eine Damenbinde, eine Wegwerfwindel oder ähnliches ist im allgemeinen mit einer flüssigkeitsdurchlässigen Decklage ausgerüstet, die einen Absorptionskern des Artikels bedeckt. Derartige Decklagen nach dem Stand der Technik umfassen typischerweise Kunststoffolien mit Öffnungen oder Kunststoffolien mit flüssigkeitsführenden Kapillarröhrchen, die von den jeweiligen Öffnungen ausgehen. Letztere ist beispielsweise im US Patent Nr. 3.929.135 und im japanischen Amtsblatt zur Veröffentlichung von Gebrauchsmusteranmeldungen Nr. 1991-63325 aufgezeigt. Eine weitere Decklage ist aus der EP-A-0 156 471 bekannt.

Im wesentlichen zielt die vorliegende Erfindung auf die Entwicklung einer verbesserten Decklage mit einem gewebe- bzw. textilartigen Erscheinungsbild ab, die mit flüssigkeitsführenden Kapillarröhrchen ausgerüstet ist, die von den jeweiligen Öffnungen ausgehen und die so eingerichtet sind, daß der Rückfluß der Körperflüssigkeiten effektiv verhindert wird, sowie auf ein Herstellungsverfahren für derartige Decklagen.

Gemäß dem vorstehend erwähnten US Patent Nr. 3.929.135 hat jedes der flüssigkeitsführenden Kapillarröhrchen die Form einer trichter- bzw. konusförmigen Kapillare. Eine derartige sich verjüngende Konstruktion der flüssigkeitsführenden Kapillarröhrchen basiert auf der Absicht, das Fließen der Körperflüssigkeiten zu einem darunter liegenden Absorptionskern hin zu erleichtern und einen eventuellen Rückfluß der Körperflüssigkeiten vom Absorptionskern zu verringern. Gemäß dem Stand der Technik ist es jedoch erforderlich, das Ende der Kapillare nicht nur teilweise, sondern vollständig in dichten Kontakt mit dem Absorptionskern zu bringen. Dieses Erfordernis kann die Menge des unerwünschten Rückflusses erhöhen. Gemäß dem japanischen Amtsblatt zur Veröffentlichung von Gebrauchsmusteranmeldungen Nr. 1991-63325 ist das untere Ende der Kapillare schräg abgeschnitten, um so eine spitz zulaufende Spitze zu bilden. Dadurch kann eine gewisse Wirkung zur Verhinderung des Rückflusses erwartet werden, wenn nur die scharf zulaufende Spitze der Kapillare mit dem Absorptionskern in Berührung gebracht wird. Bedauerlicherweise ist kein bestimmtes Verfahren zur Bildung einer scharf zulaufenden Spitze der Kapillare aufgezeigt. Darüber hinaus ist dieser Vorschlag nicht praktisch anwendbar, da das flüssigkeitsführende Kapillarröhrchen allgemein zu fein ist, um ohne weiteres zur Bildung der gewünschten scharf zulaufenden Spitze abgeschnitten werden zu können. Darüber hinaus sind die meisten Verteilungsmuster der Öffnungen auf bekannten Kunststoffolien geometrisch, beispielsweise kreisförmig, elliptisch, gleichseitig dreieckig und ähnliches, was in nachteilhafter Weise zu einem zu kunststoffartigen Erscheinungsbild führt, das weit von einem gewebe- oder stoffartigen Erscheinungsbild entfernt ist, das für einen absorbierenden Hygieneartikel wünschenswert ist.

Demgemäß ist es eine Hauptaufgabe der Erfindung, eine verbesserte Decklage zur Verwendung in absorbierenden Hygieneartikeln aufzuzeigen, die Öffnungen aufweist, die von einer Kombination von geraden und gekrümmten Linien begrenzt sind, um so ein textil- bzw. gewebeartiges Erscheinungsbild zu erzeugen, sowie ein Herstellungsverfahren für eine derartige Decklage unter Verwendung einer Form für Kunststoffolien aufzuzeigen, wobei die Form mit asymmetrisch angeordneten Öffnungen versehen ist, so daß das textilähnliche Erscheinungsbild wie auch die flüssigkeitsführenden Kapillarröhrchen, die von den jeweiligen oberen Öffnungen ausgehen und in schräg abgeschnittenen unteren Öffnungen enden, ohne weiteres hergestellt werden können.

### BESCHREIBUNG DER ERFINDUNG

Vorstehend dargelegte Aufgabe wird nach einem ersten Aspekt der Erfindung durch eine Decklage zur Verwendung in einem absorbierenden Hygieneartikel gelöst, die in einer oberen Fläche der Lage ausgebildete obere Öffnungen, von den jeweiligen oberen Öffnungen abwärts sich erstreckende flüssigkeitsführende Kapillarröhrchen und an den unteren Enden der jeweiligen flüssigkeitsführenden Kapillarröhrchen gebildete untere Öffnungen aufweist, wobei die Konfiguration in der Ebene jeder oberen Öffnung von einer Kombination von geraden und gekrümmten Linien gebildet ist, jeweils zwei benachbarte obere Öffnungen voneinander durch eine Rippe mit einer Breite von 0,1 bis 3 mm getrennt sind, jede obere Öffnung eine Fläche von 0,1 bis 25 mm² aufweist, jede untere Öffnung sich bezüglich der Längsrichtung des zugehörigen flüssigkeitsführenden Kapillarröhrchens schräg öffnet und jede untere Öffnung von einer unregelmäßig wellenförmig ausgebildeten Umfangskante begrenzt ist.

Gemäß einem zweiten Aspekt der Erfindung wird die vorstehend dargelegte Aufgabe durch ein Herstellungsverfahren für die obere Lage gelöst, das in Schritten das Einbringen einer thermoplastischen Folie in eine Form mit einem diese durchbrechenden Öffnungsmuster und das Erwärmen der Folie unter Druckluft zur Bildung von flüssigkeitsführenden Kapillarröhrchen umfaßt, wobei jede die Form durchbrechend ausgebildete Öffnung eine ebene Konfiguration aufweist, die asymmetrisch ist oder eine Symmetrieachse hat. Vorzugsweise ist die ebene Konfiguration jeder Öffnung der Form polygonal.

Bei der erfindungsgemäßen Decklage sind obere Öffnungen jeweils von einer Kombination von geraden und gekrümmten Linien begrenzt, so daß sich eine unregelmäßige Konfiguration bildet, und je zwei benachbarte obere Öffnungen sind voneinander durch eine feine Rippe getrennt, was zu einem unregelmäßigen Öffnungsmuster führt, das ein textilartiges Erscheinungsbild der gesamten Lage schafft.

Gemäß dem erfindungsgemäßen Verfahren ist die Öffnungsanordnung auf der Form asymmetrisch oder liniensymmetrisch, so daß die Folie unter Druckluft an Stellen, wo sie gegen die Umfangskanten der jeweiligen oberen Öffnungen vorgespannt ist, aufgebläht wird und platzt, wodurch die jeweiligen flüssigkeitsführenden Kapillarröhrchen gebildet werden. Die Wände der flüssigkeitsführenden Kapillarröhrchen sind entlang den Umfangskanten, die nahe der Berststelle der Folie liegen, relativ kurz, und entlang den Umfangskanten, die von der Berststelle der Folie entfernt angeordnet sind, relativ lang. Damit ist am unteren Ende jedes Kapillarröhrchens durch dessen Wand eine untere Öffnung gebildet, die sich schräg nach unten öffnet. Durch das Zerplatzen bzw. Zerbersten der Folie weist die Umfangskante dieser unteren Öffnung eine unregelmäßig gezackte Form auf.

### KURZE BESCHREIBUNG DER ZEICHNUNG

Die Erfindung wird im Detail unter Bezug auf die beiliegenden Figuren erläutert. Es zeigen:
- Fig. 1: eine teilweise ausgebrochene perspektivische Darstellung einer Damenbinde;
- Fig. 2: einen Ausriß aus der Decklage in Draufsicht und Schnittdarstellung im vergrößerten Maßstab;
- Fig. 3: eine schematische Darstellung der Art und Weise, in der die Decklage gebildet wird;
- Fig. 4: Beispiele von durch die Form gebildeten Öffnungen in Draufsicht und Schnittdarstellung; und
- Fig. 5: eine Draufsicht auf eine Variante der in der Form ausgebildeten Öffnungen;

### BEVORZUGTE AUSFÜHRUNGSFORMEN DER ERFINDUNG

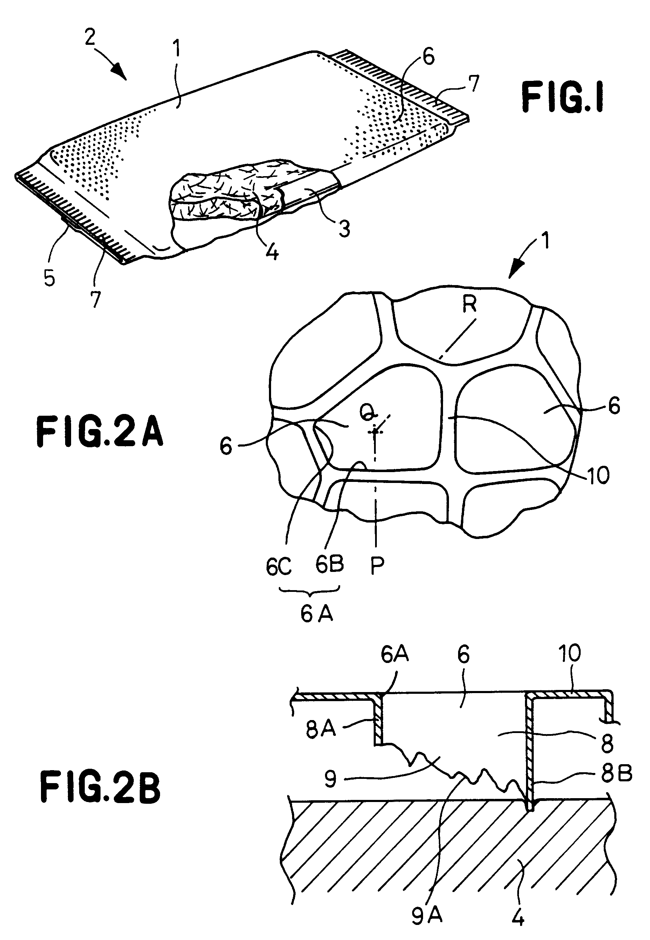

Fig. 1 ist eine perspektivische Darstellung einer Damenbinde 2, bei der eine Decklage 1 gemäß der Erfindung verwendet ist. Die Darstellung ist teilweise ausgebrochen. Die Binde 2 verfügt über die flüssigkeitsdurchlässige Decklage 1, eine flüssigkeitsundurchlässige untere Lage 3 und einen Absorptionskern 4, der zwischen diese beiden Lagen gelegt ist. Die Decklage 1 ist aus thermoplastischer Folie hergestellt und bedeckt den Absorptionskern 4 sowie die untere Lage 3. Die in Querrichtung gegenüberliegenden Ränder der Decklage 1 sind an der Rückseite der Binde überlappt und anschließend durch eine Siegellinie 5 miteinander verbunden. Die in Längsrichtung gegenüberliegenden Ende der Binde 2 sind durch Siegellinien 7 verschlossen. Die Decklage 1 weist an ihrer Oberseite eine Vielzahl von oberen Öffnungen 6 auf. Die untere Lage 3 dient dazu, das Austreten von Flüssigkeit zu verhindern.

Fig. 2A zeigt eine vergrößerte Teildarstellung der Decklage 1 mit der Anordnung der oberen Öffnung 6 in der Ebene und Fig. 2B ist eine Schnittdarstellung entlang der Linie PQR, wobei der Punkt Q einem Schwerpunkt G entspricht, wie weiter unten erläutert wird. Die obere Öffnung 6 ist von einem unregelmäßig geformten Umfangsrand 6A umgeben, der durch eine Kombination von geraden und gekrümmten Linien gebildet wird, und jeweils zwei benachbarte obere Öffnungen sind voneinander durch eine feine Rippe 10 getrennt. Ein flüssigkeitsführendes Kapillarröhrchen 8 erstreckt sich von jeder oberen Öffnung zur Unterseite der Decklage 1 und endet in einer unteren Öffnung 9. Das flüssigkeitsführende Kapillarröhrchen 8 hat an einer Seite einen relativ kurzen Wandabschnitt 8A und an der gegenüberliegenden Seite einen relativ langen Wandabschnitt 8B. Diese Wandabschnitte 8A, 8B gehen ineinander über und bilden so die bezüglich zur Längsrichtung des flüssigkeitsführenden Kapillarröhrchens 8 schräge untere Öffnung 9. Die untere Öffnung 9 ist von einem Umfangsrand 9A umgeben, der faser-, haken- oder sägezahnartig oder in ähnlicher Weise unregelmäßig gezackt ist. Das untere Ende des relativ langen Wandabschnittes 8B berührt den Absorptionskern 4 und bildet damit eine Brücke. Jede obere Öffnung 6 hat eine Fläche von 0,1 bis 25 mm². Die oberen Öffnungen können einzelne unregelmäßige Formen aufweisen oder können eine Kombination von regelmäßigen Formen darstellen, die jeweils in relativ langen Abständen aufscheinen, so daß die Anordnung dieser oberen Öffnungen 6 kein einfaches geometrisches Muster bildet. Die Rippe 10 hat eine Breite von 0,1 bis 3 mm und die Wand 8 des Kapillarröhrchens erstreckt sich über 0,3 bis 7 mm zwischen der oberen und unteren Öffnung des flüssigkeitsführenden Kapillarröhrchens. Die oberen Öffnungen 6 und ihre Anordnung in der Weise, daß jeweils zwei benachbarte obere Öffnungen mit ihren jeweiligen unregelmäßigen Formen voneinander durch die feine Rippe 10 getrennt sind, schaffen das gewünschte Gewebe- bzw. textilähnliche Erscheinungsbild, das von einer herkömmlichen Decklage mit einem geometrischen Anordnungsmuster der oberen Öffnungen nicht erzielbar ist. Falls erwünscht können die Rippen 10 einer Kräuselungs- oder Verfilzungsbehandlung unterzogen werden, um eine weitere Verbesserung des textilähnlichen Erscheinungsbildes zu erzielen.

Wie aus Fig. 1 und 2 ersichtlich ist, fließen beim Gebrauch der Decklage 1 abgegebene Körperflüssigkeiten in die oberen Öffnungen 6 und werden anschließend durch die entlang den Innenwänden der flüssigkeitsführenden Kapillarröhrchen 8 auftretende Kapillarwirkung abwärts geführt, worauf sie sich über die durch die unteren Enden der Wandabschnitte 8B der Kapillarröhrchen in Berührung mit dem Absorptionskern 4 gebildeten Brücken von selbst im Absorptionskern 4 verteilen. Ein Rückfluß vom Absorptionskern 4 muß notwendigerweise entlang den Wandabschnitten 8B der Kapillarröhrchen auftreten, die mit dem Absorptionskern 4 in Berührung stehen, so daß daher die Menge eines derartigen Rückflusses im Vergleich zum Stand der Technik wesentlich reduziert ist, wo die gesamte Umfangskante jeder flüssigkeitsführenden Kapillare an deren unterem Ende vollständig mit dem flüssigkeitsabsorbierenden Kern in Berührung gebracht wurde. Wirkt das Körpergewicht des Benutzers auf die Binde 2, so können die Wandabschnitte 8B der Kapillarröhrchen und die benachbarten Bereiche nach innen umklappen. Die Zahnung bzw. wellenförmige Ausbildung der Umfangsränder 9A dient jedoch dazu, die vollständige Blockierung der jeweiligen unteren Öffnungen 9 zu verhindern, so daß gewisse Lücken zwischen dem Umfangsrand 9A und dem Absorptionskern 4 verbleiben und die Körperflüssigkeiten in den Absorptionskern 4 fließen können. Zusätzlich erleichtert die Zahnung bzw. wellenförmige Ausbildung der Umfangsränder 9A die Verankerung der Decklage 1 am Fasermaterial des Absorptionskernes 4, wodurch verhindert wird, daß die Decklage 1 und der Absorptionskern 4 relativ zueinander verschoben werden und Einschränkungen des Tragekomforts während der Benutzung der Binde 2 vermieden werden.

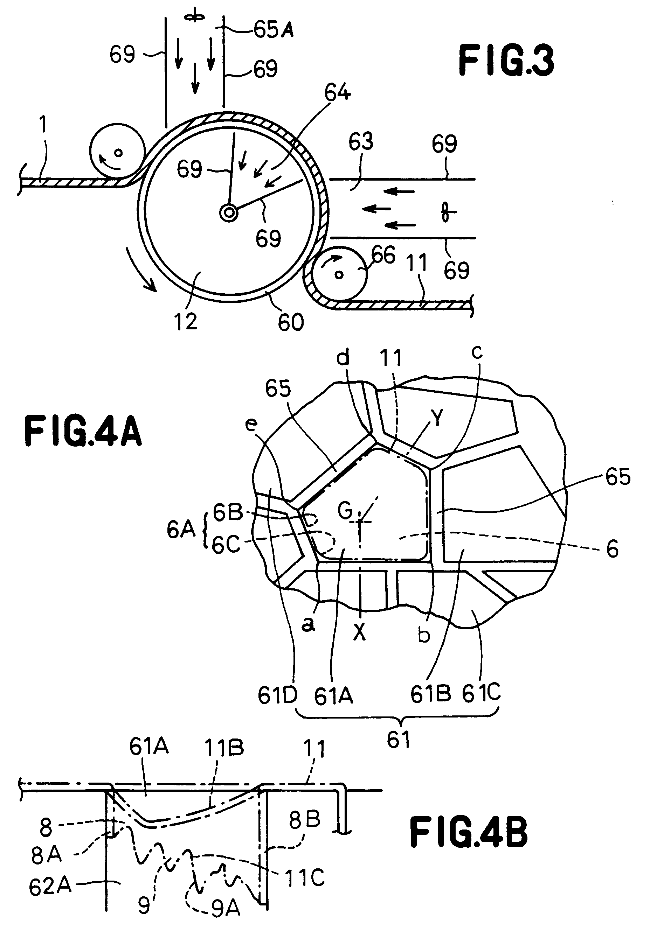

Fig. 3 zeigt schematisch ein Formverfahren zur Herstelllung der Decklage 1. Eine.Ausgangsfolie 11 für die Decklage 1 besteht aus thermoplastischem Material und wird kontinuierlich einer Formwalze 12 zugeführt, die an ihrem Umfang eine umlaufende Form 60 trägt. Die Form 60 umfaßt einen Zylinder mit einer glatten Umfangsfläche, in dem diesen durchbrechend ein Öffnungsmuster ausgebildet ist, das eine Vielzahl von später beschriebenen Öffnungen 61 umfaßt, sowie radial von den jeweiligen Öffnungen 61 zur Walzenmitte verlaufende Kapillarröhrchen 62. Die Formwalze 12 hat einen Saugabschnitt 64, der einem Sektor der Form 60 entspricht, unter dem eine Vakuumquelle angeordnet ist, einen Heizabschnitt 63, der einem in Laufrichtung vor dem Saugabschnitt 64 liegenden Abschnitt der Form 60 entspricht, über dem ein Warmluftgebläse angeordnet ist, das auf die Ausgangsfolie 11 gerichtet ist, sowie einen Abkühlabschnitt 65A, der einem dem Saugabschnitt 64 folgenden Abschnitt der Form 60 entspricht, über dem ein Kaltluftgebläse auf die Ausgangsfolie 11 gerichtet ist. Die jeweiligen Abschnitte 63, 64 und 65A sind durch Trennwände 69 getrennt. Die Ausgangsfolie 11 wird von einer Führungswalze 66 auf die Form 60 geführt und anschließend in dichter Berührung mit der Form 60 gegen den Uhrzeigersinn in Umlauf gebracht. Während dieses Umlaufes wird die Ausgangsfolie 11 von Warmluft erweicht, wenn sie den Heizabschnitt 63 durchläuft, und tritt in den Saugabschnitt 64 ein, in dem die Ausgangsfolie 11 in die jeweiligen Kapillarröhrchen 62 unter der Wirkung des Vakuums ausgebeult wird, wie weiter unten beschrieben werden wird, bis die jeweiligen unteren Enden der Aufblähungen zerbersten. Damit werden beim Abkühlen der Ausgangsfolie 11 im Kühlbereich 65 die oberen Öffnungen 6, deren Gestalt im wesentlichen mit den Öffnungen 61 der Form übereinstimmt, und die zugehörigen flüssigkeitsführenden Kapillarröhrchen 8 wie auch die unteren Öffnungen 9 geformt, die von den unteren Enden der jeweiligen flüssigkeitsführenden Kapillarröhrchen begrenzt sind, die im vorhergehenden Saugabschnitt 64 zerborsten sind. Anschließend wird die vakuumgeformte Folie von der Form abgenommen und in Abschnitte mit der für die einzelnen Decklagen 1 erforderlichen Größe getrennt.

Fig. 4A zeigt eine Draufsicht auf die öffnungen, die allgemein mit Bezugszeichen 61 bezeichnet sind, und individuelle Öffnungen 61A, 61B, 61C ... umfassen, die zusammen das Öffnungsmuster bilden. Fig. 4B ist eine Schnittdarstellung der Öffnung 61A entlang einer Linie XGY und zeigt ein Kapillarröhrchen 62A, das dem allgemeinen Bezugszeichen 62 entspricht. Jede Öffnung 61 hat eine polygonale asymmetrische Anordnung mit einem Schwerpunkt (Bildmittelpunkt) G. Die Öffnung 61A ist beispielsweise durch ein asymmetrisches Fünfeck a b c d e gebildet, wobei der Schwerpunkt nahe der Seite a e und entfernt von der Seite c d des Fünfeckes angeordnet ist. Die jeweiligen Öffnungen 61A, 61B, 61C ... sind voneinander durch jeweilige Rippen 65 getrennt. Die Kapillarröhrchen 62, die von den jeweiligen Öffnungen 61 ausgehen, sind am unteren Ende offen und der Vakuumwirkung im Saugabschnitt 64 ausgesetzt.

In Fig. 4A und 4B ist die Art und Weise dargestellt, in der die Ausgangsfolie 11 dem Formverfahren unterzogen wird, durch das in der Ausgangsfolie 11 durch Vakuumformung die oberen und unteren Öffnungen 6, 9 und die flüssigkeitsführenden Kapillarröhrchen 8 ausgebildet werden. In Fig. 4A ist der Umfangsrand 6A der oberen Öffnung 6, der im wesentlichen gemäß der Form der Öffnung 61A ausgebildet wird, durch eine unterbrochene Linie angedeutet, und in Fig. 4B ist ein aufgeblähter Zustand 11B und ein zerborstener Zustand 11C der Ausgangsfolie 11 ebenfalls jeweils durch unterbrochene Linien dargestellt. Die Abschnitte der Ausgangsfolie 11, die mit den Seiten a b, c d, ... des Fünfeckes in Berührung gekommen sind, werden als gerade Linien 6B ausgebildet, die Abschnitte der Ausgangsfolie 11, die jedoch mit den Ecken a, b, c, ... in Berührung gekommen sind, werden als gekrümmte Linien 6C ausgebildet, da diese Abschnitte nicht in Übereinstimmung mit der Form bringbar sind. Folglich umfaßt der gesamte Umfangsrand 6A eine Kombination der geraden und gekrümmten Linien 6B, 6C, das heißt, daß es schwierig ist, die jeweiligen oberen Öffnungen 6 mit den entsprechenden Öffnungen 61 der Form in formmäßige Übereinstimmung zu bringen. Diese geraden und gekrümmten Linien verändern sich wesentlich in Abhängigkeit auch von geringen Veränderungen der Stärke der Ausgangsfolie, wie auch der Formungsbedingungen, und dieselbe Öffnung 61 führt oftmals zu ungleichmäßigen, unbestimmten oberen Öffnungen 6. Die Ausbeulung der Ausgangsfolie 11 beginnt an einem dem Schwerpunkt G jeder Öffnung 61 entsprechenden Ort, worauf die Ausgangsfolie 11 an dieser Stelle zerbirst und damit das flüssigkeitsführende Kapillarröhrchen 8 und die untere Öffnung 9 bildet. Bezüglich der Öffnung 61A ist beispielsweise der an der Seite a e gebildete Kapillarwandabschnitt 8A relativ kurz und der an der Seite c d angrenzend gebildete Kapillarwandabschnitt 8B relativ lang. Die Kapillarwandabschnitte 8A, 8B sind koninuierlich miteinander verbunden, so daß die untere Öffnung 9 bezüglich der Längsrichtung des flüssigkeitsführenden Kapillarröhrchens 8 schräg ausgerichtet sind. Der Umfangsrand 9A der unteren Öffnung 9 hat eine unregelmäßige Wellenform, wie beispielsweise faser-, haken- oder sägezahnähnliche Unregelmäßigkeiten, die durch das Zerbersten der Ausgangsfolie 11 entstehen.

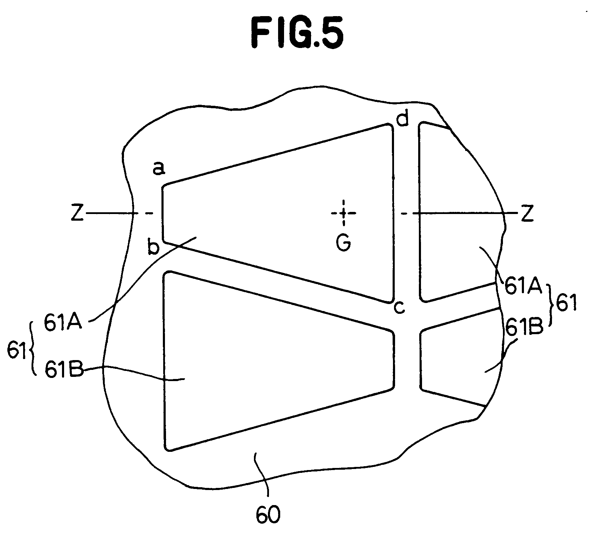

Fig. 5 zeigt eine Draufsicht auf Öffnungen 61, die jeweils nur bezüglich einer Achse Z-Z symmetrisch sind. Beispielsweise ist die Öffnung 61A als Trapez a b c d ausgebildet, dessen Bildmittelpunkt auf der Symmetrieachse Z-Z näher an der Seite c d als an der Seite a b liegt. Durch eine derartige Konfiguration der Öffnungen ist es möglich, eine Decklage 1 zu erhalten, deren untere Öffnungen der flüssigkeitsführenden Kapillarröhrchen unregelmäßig in Wellenform verlaufende Ränder 9A aufweisen.

Während die Konfiguration der Öffnungen 61in der Ebene nicht auf Vielecke beschränkt ist, sondern auch nur durch gekrümmte Linien gebildet sein kann, wird es in diesem Fall schwierig sein, die entsprechende obere Öffnung 6 als Kombination von geraden und gekrümmten Linien auszubilden. Es sei angemerkt, daß die Öffnungen 61 weder Figuren mit zwei oder mehr Symmetrieachsen noch punktsymmetrische Figuren, z.B. Kreis, Ellipse und gleichseitige Vielecke umfassen. Sind die Öffnungen 61 kreisförmig, so beginnt das Anschwellen bzw. die Ausbeulung der Ausgangsfolie 11 im Mittelpunkt der jeweiligen Öffnung 61 und das Zerbersten erfolgt an diesem Punkt, wodurch ein flüssigkeitsführendes Kapillarröhrchen gebildet wird, dessen Wand am gesamten Umfang eine im wesentliche gleiche Länge aufweist, wobei die untere Öffnung nicht schräg verläuft. Sind die Öffnungen 61 elliptisch geformt, so beginnt die Ausbeulung der Ausgangsfolie 11 an einem Kreuzungspunkt der Haupt- und Nebenachsen jeder Öffnung 61, worauf das Zerbersten an diesem Punkt folgt und das flüssigkeitsführende Kapillarröhrchen 8 mit relativ langen Wänden an den gegenüberliegenden Enden der Hauptachse und relativ kurzen Wänden an den gegenüberliegenden Enden der Nebenachse ausgebildet wird.

Die Decklage 1 kann aus thermoplastischem Material, wie etwa Polyethylen, Polypropylen oder ähnlichem mit einer Stärke von 0,01 bis 0,2 mm hergestellt werden.

Die Form 60 kann durch Elektroformung hergestellt sein.

Die gemäß dem erfindungsgemäßen Verfahren aus thermoplastischem Material hergestellte Decklage weist obere Öffnungen auf, die jeweils von einer Kombination von geraden und gekrümmten Linien gebildet sind, um so eine unbestimmte Form herzustellen, wobei die Öffnungen voneinander durch feine Rippen getrennt sind. Durch diese Anordnung wird bei der erfindungsgemäßen Decklage das gewünschte gewebeartige Erscheinungsbild erzielt. Jedes der flüssigkeitsführenden Kapillarröhrchen endet in einer unteren Öffnung, die sich bezüglich der Längsrichtung des flüssigkeitsführenden Kapillarröhrchens schräg nach unten öffnet, und die unterste Spitze des Kapillarröhrchens wird mit dem Absorptionskern in Berührung gebracht, um die den Rückfluß verhindernde Wirkung der Kapillarröhrchen zu verbessern. Beim erfindungsgemäßen Verfahren zur Herstellung der Decklage wird eine Form verwendet, die ein Öffnungsmuster aufweist, das eine Vielzahl von asymmetrischen Vielecken oder von Vielecken, die jeweils ein einzelne Symmetrieachse aufweisen, umfaßt, so daß die Decklage mit einem textilartigen Erscheinungsbild hergestellt werden kann und die untere Öffnung jedes flüssigkeitsführenden Kapillarröhrchens sich schräg öffnen kann. Der wellenförmige Verlauf des unteren Umfangsrandes der unteren Öffnung dient dazu, das vollständige Blockieren der unteren Öffnung zu verhindern, auch wenn das flüssigkeitsführende Kapillarröhrchen unter dem Körpergewicht des Trägers zusammengedrückt wird.

## Patentansprüche

1. Decklage (1) zur Verwendung in einem absorbierenden Hygieneartikel mit in einer Oberfläche ausgebildeten oberen Öffnungen (6), flüssigkeitsführenden Kapillarröhrchen (8), die sich von den jeweiligen oberen Öffnungen (6) nach unten erstrecken, und unteren Öffnungen (9), die von den unteren Enden der jeweiligen flüssigkeitsführenden Kapillarröhrchen (8) gebildet werden, wobei die ebene Konfiguration jeder der oberen Öffnungen (6) durch eine Kombination von geraden und gekrümmten Linien gebildet ist, jeweils zwei benachbarte obere Öffnungen (6) voneinander durch eine Rippe (10) mit einer Breite von 0,1 bis 3 mm getrennt sind und jede obere Öffnung (6) eine Fläche von 0,1 bis 25 mm² aufweist, und jede untere Öffnung von einem unregelmäßig wellenförmig verlaufenden Umfangsrand umgeben ist,
**dadurch gekennzeichnet,**
daß jede untere Öffnung (9) sich bezüglich der Längsrichtung des zugehörigen flüssigkeitsführenden Kapillarröhrchens schräg öffnet.

2. Herstellungsverfahren für die Decklage (1) nach Anspruch 1,
umfassend in Schritten das Auflegen einer thermoplastischen Folie (11) auf eine Form (60), die ein die Form (60) durchbrechendes Öffnungsmuster aufweist, und das Erwärmen dieser Folie (11) unter Druckluft, so daß den Öffnungen (61A,61B,61C) entsprechende Teile der Folie (11) sich in Kapillarröhrchen (62A) aufblähen, die von den jeweiligen Öffnungen (61A,61B,61C) nach unten zur Unterseite der Form (11) verlaufen, und anschließend zerbersten, was zur Bildung der oberen Öffnungen (6) entsprechend dem Öffnungsmuster, der flüssigkeitsführenden Kapillarröhrchen (8), die von den oberen Öffnungen (6) nach unten verlaufen und der sich schräg an den unteren Enden der flüssigkeitsführenden Kapillarröhrchen (8) öffnenden unteren Öffnungen (9) führt.

3. Herstellungsverfahren nach Anspruch 2,
wobei die ebene Konfiguration jeder Öffnung (61A,61B,61C), die durch die Form (11) gebildet wird, eine einzige Symmetrieachse aufweist.

4. Herstellungsverfahren nach Anspruch 2 oder 3, wobei die ebene Konfiguration jeder der durch die Form gebildeten Öffnungen (61A,61B,61C) polygonal ist.

## Claims

1. A cover layer (1) for use in an absorbent sanitary article, with upper openings (6) formed in one surface, liquid conducting capillary tubes (8) which extend down from the respective upper openings (6), and lower openings (9) which are formed by the lower ends of the respective liquid conducting capillary tubes (8), wherein the configuration in the plane of each of the upper openings (6) is formed by a combination of straight and curved lines, each pair of adjacent openings is separated the one from the other by a web (10) with a width of 0.1 to 3 mm and each upper opening (6) has an area of 0.1 to 25 mm², and each lower opening is surrounded by an irregularly wavy peripheral edge, characterized in that each lower opening (9) opens obliquely with respect to the longitudinal direction of the corresponding liquid conducting capillary tube.

2. A method of making the cover layer (1) according to claim 1, comprising the steps of laying a thermoplastic film (11) on a mould (60) which has of a pattern of openings passing through the mould (60), and heating this film under air pressure, so that parts of the film (11) corresponding to the openings (61A, 61B, 61C) blow out into capillary tubes (62A), which run from the respective openings down to the underside of the mould (11), and then burst, which leads to the formation of the upper openings (6) corresponding to the pattern of openings, the liquid conducting capillary tubes (8) which run down from the upper openings (6) and the lower openings (9) opening obliquely at the lower ends of the liquid conducting capillary tubes (8).

3. A method of manufacture according to claim 2, wherein the configuration in the plane of each opening (61A, 61B, 61C) formed by the mould (11) has a single axis of symmetry.

4. A method of manufacture according to claim 2 or 3, wherein the configuration in the plane of each opening (61A, 61B, 61C) formed by the mould is polygonal.

## Revendications

1. Couche de couverture (1) pour utilisation dans un article hygiénique absorbant, comportant des orifices supérieurs (6) pratiqués dans une surface, des tubules capillaires (8) servant à guider les liquides, qui se dirigent vers le bas, en partant des orifices supérieurs (6), et des orifices inférieurs (9) constitués par les extrémités inférieures des différents tubules capillaires (8) conduisant les liquides, la configuration plane de chacun des orifices supérieurs (6) étant formée par une combinaison de lignes droites et courbes, deux orifices supérieurs (6) voisins étant à chaque fois séparés l'un de l'autre par une nervure (10) d'une largeur de 0,1 à 3 mm, et chaque orifice supérieur (6) présentant une aire de 0,1 à 25 mm², et chaque orifice inférieur étant délimité par un bord périphérique à profil ondulant irrégulier, caractérisée en ce que chaque orifice inférieur (9) s'ouvre en oblique par rapport au sens longitudinal du tubule capillaire associé, servant à conduire les liquides.

2. Procédé de fabrication de la couche de couverture (1) selon la revendication 1, comprenant, successivement, la pose d'un film thermoplastique (11) sur une forme ajourée (60) présentant des orifices traversant ladite forme (60), et le chauffage de ce film (11) sous air comprimé, en sorte que des parties du film (11) correspondant aux orifices (61A,61B,61C) s'enflent en tubules capillaires (62) qui se dirigent vers le bas, partant des orifices respectifs (61A,61B,61C), en direction de la face inférieure de la forme (60), et crèvent, ce qui conduit à la formation des orifices supérieurs (6), conformément au motif d'ajourage, des tubules capillaires (8) servant à conduire les liquides, qui se dirigent vers le bas, partant des orifices supérieurs (6), et des orifices inférieurs (9) qui s'ouvrent en oblique aux extrémités inférieures des tubules capillaires (8) servant à conduire les liquides.

3. Procédé de fabrication suivant la revendication 2, selon lequel la configuration plane de chaque orifice (61A,61B,61C), formé par la forme (60), présente un seul axe de symétrie.

4. Procédé de fabrication suivant l'une des revendications 2 ou 3, selon lequel la configuration plane des orifices (61A,61B,61C), formés par la forme, est polygonale.
